Europäisches Patentamt

European Patent Office

Office européen des brevets

(11) Publication number: **0 388 109 B1**

# EUROPEAN PATENT SPECIFICATION

(45) Date of publication of patent specification: **15.03.95**  (51) Int. Cl.6: **A01H 4/00**

(21) Application number: **90302587.2**

(22) Date of filing: **12.03.90**

(54) Potato Production.

(30) Priority: **11.03.89 KR 893009**

(43) Date of publication of application:
**19.09.90 Bulletin 90/38**

(45) Publication of the grant of the patent:
**15.03.95 Bulletin 95/11**

(84) Designated Contracting States:
**AT BE CH DE DK ES FR GB GR IT LI LU NL SE**

(56) References cited:
**EP-A- 293 488**
**WO-A-88/02213**
**WO-A-88/04137**
**FR-A- 2 610 785**

**Annuals of Botany, vol. 48, 1981, pages 787-796; G. hussey & N.J. Stacey: "In vitro propagation of potato (Solanum tuberosum L.)"**

**"Potato Physiology" 1985 Academic Press Chapter 15 P-J. Wang & C. Hu: "Potato tissue culture and its applications in agriculture" pages 503-577**

(73) Proprietor: **KOREA INSTITUTE OF SCIENCE AND TECHNOLOGY**
**39-1 Haweolgog-Dong,**
**Sungbuk-Gu**
**Seoul (KR)**

(72) Inventor: **Joung, Hyouk**
**39-1 Haweolgog-dong,**
**Sungbuk-gu**
**Seoul (KR)**
Inventor: **Liu, Jang-Ryol**
**39-1 Haweolgog-dong,**
**Sungbuk-gu**
**Seoul (KR)**
Inventor: **Hong, Joo-Bong**
**39-1 Haweolgog-dong,**
**Sungbuk-gu**
**Seoul (KR)**
Inventor: **Yang, Seung-Gyun**
**39-1 Haweolgog-dong,**
**Sungbuk-gu**
**Seoul (KR)**
Inventor: **Lee, Haeng-Soon**
**39-1 Haweolgog-dong,**
**Sungbuk-gu**
**Seoul (KR)**

Rank Xerox (UK) Business Services
(3. 10/3.09/3.3.3)

Potato Research, vol. 16, 1973, pages 73-79; L. Garcia Torres et al: "In vitro tuberitation of patato sprouts as affected by ethrel and giberallic acid"

P.V. Ammirato et al -ed- "Handbook of plantcell culture" Vol. 3 Crop Species Chapter 11, S.A. Miller & L. Lipschutz: "Potato" pages 291-326

"In vitro plant tissue culture and its agricultural application" 1986, Butterworths, Chapter 11A, A.J. Abbott & A.R. Belcher:"Potato tuber formation in vitro" pages 113-122

Inventor: **Jeon, Jae-Heung**
**39-1 Haweolgog-dong,**
**Sungbuk-gu**
**Seoul (KR)**
Inventor: **Koo, Jeong-Sook**
**39-1 Haweolgog-dong,**
**Sungbuk-gu**
**Seoul (KR)**

(74) Representative: **Perry, Robert Edward et al**
**GILL JENNINGS & EVERY**
**Broadgate House**
**7 Eldon Street**
**London EC2M 7LH (GB)**

## Description

Field of the Invention

This invention relates to the production of pathogen-free (especially virus-free) seed potatoes (potato microtubers) by plant tissue culture.

Background of the Invention

The potato belongs to the Solanaceae family and is characterised by its vegetative propagation by means of tubers. One of the most serious problems commonly found in most crops which undergo vegetative propagation, and especially in the case of the potato, is reduction in yield caused by virus infection.

It is well known that most virus infections are caused by aphids. In order to produce virus-free seed potato, the seed potato production area may therefore be in a relatively cold location, but yields are then low.

Recently, due to the rapid development of plant tissue culture techniques, it has been possible to mass-propagate many kinds of plant in vitro. In the case of the potato, rapid propagation of virus-free plantlets using the shoot-tip culture technique is well established; see Goodwin et al (1980) Potato Res. 23:9-23; Hussey et al (1981) Ann. Bot. 48:787-796; and Roset et al (1976) Potato Res. 19:173-178. However, the transplanting process from the in vitro system to soil is a time- and labour-consuming process; further, many of the delicate potato shoots cannot survive the sudden change of growth environment.

There have been several reports about the formation of potato microtubers in vitro, but the production efficiency was so poor that the phenomenon of microtuberisation has been only an experimental tool for the study of the tuberisation physiology of the potato; see Garcia-Torres et al (1973) Potato Res. 16:73-79; Abbott et al (1986) "In Vitro Plant Tissue Culture and its Agricultural applications" Butterworths, pp. 113-122; and Hussey et al (1984) Ann. Bot. 53:565-578.

Recently, there have been attempts to produce virus-free, good-quality seed potatoes in large quantities, by planting microtubers produced in vitro by liquid culture; see Wang et al (1982) Amer. Potato Jour. 59:33-39; and International Patent Application No. PCT/HU86/00053. However, the production efficiency of microtubers using these methods is still too low to replace natural seed potatoes. Moreover, the microtubers produced by these liquid culture methods readily undergo desiccation during storage; they frequently vitrify and are thus unusable as artificial seed potatoes. In spite of these problems, potato microtubers which are formed during tissue culture of potato shoots are a substitute for potato shoot tips, because potato microtubers are much less delicate and easier to handle at the transplanting stage than tissue culture-produced plantlets. Nevertheless, in consideration of the characteristic feature of vegetative propagation of the potato, the quantity of seed potatoes required annually is so enormous that, even if microtubers should prove practical, they will be effectively insignificant unless some means is developed to mass-produce an enormous quantity of microtubers in a small space and thus provide them at low cost.

FR-A-2 610 785 discloses a potato propagation process, in which shoots are induced to proliferate on a liquid or solid medium, and then on fresh medium when roots are present, as in the preamble of claim 1.

An object behind the present invention is to overcome these disadvantages.

Summary of the Invention

The invention is defined by the claims. As a result of this invention, it is possible to mass-produce artificial seed potatoes more than 30 times as efficiently as by known methods of microtuber production.

Detailed Description of the Invention

The present invention provides a process for mass production of artificial seed potatoes, comprising several steps. The following Examples illustrate them; certain results are shown in the accompanying drawings.

Example 1

Virus-free potatoes of the "Superior" species were obtained from the Horticultural Experiment Station of the Rural Development Administration, Korea. They were washed clean in running tap water, soaked in 70% ethyl alcohol for 3 minutes, surface-sterilised with 20% Chlorox (commercial) for 10 minutes, and finally seeded in square pots containing autoclaved soil (vermiculite:perlite = 1:1). After approximately one week, germination of tubers was observed in a growth chamber under a 16 hour photoperiod regime, at a constant culture temperature (25°C).

After two weeks, sprouts were 50-100 mm long on average; shoot tips (10-20 mm long) were cut off and used as the basic material for shoot tip culture. The cut shoots were washed 3 times in sterile distilled water, soaked in 70% ethyl alcohol for 30 seconds, surface-sterilised with 10% Chlorox for 10 minutes, and finally inoculated on liquid or solid medium (see Tables 1 and 2) for microtuberogenic shoot induction and proliferation.

The conditions in the growth chambers were identical to those at the time of the mother tuber germination. One week after inoculation under this environment, axillary shoots began to appear and, in most cases, they grew so rapidly as to need sub-culturing after 3-4 weeks. At that time, in vitro layering was employed in order to stimulate the induction of axillary shoots maximally, but in the case of flasks or test tubes, the technique requires so much expertise that, in this experiment, shoots were cultures in flat Petri dishes (diameter 100 mm, height 15 mm), thus inducing in vitro layering automatically, resulting in a remarkable increase in the number of axillary shoots (see Table 3). In general, the growth rate of shoots in liquid culture was superior to that of solid culture. However, after several sub-cultures using the liquid culture, shoot degeneration or vitrification due to excessive absorption of moisture frequently occurred, preventing normal growth of the potato shoots in vitro. As a result, liquid medium was used only in the initial stage and, thereafter, solid medium was mainly employed.

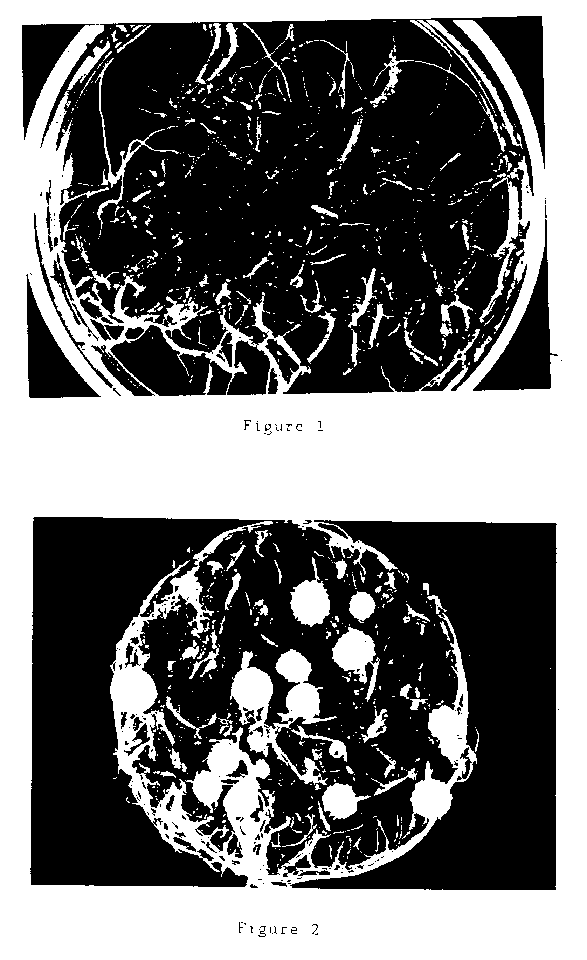

It is to be noted that not all shoots propagated on artificial culture have the ability to form microtubers when transferred under the next stage of microtuberogenic conditions, but only those with unique features, i.e. slightly elongated internodes with sessile leaves, and vigorous roots; those with hooked shoot tips (see Figure 1) especially are capable of producing microtubers in good yield (see Table 5). We have termed shoots with such specific features as "microtuberogenic shoots". "Superior" plant cell lines having this characteristic have been deposited at the Korean Type Culture Collection (deposit date 25.01.89; accession number KCTC 8445P) and at the American Type Culture Collection (deposit date 09.03.90; accession number ATCC 40769). Once formed, they retained their microtuberogenic characteristics even after 24 successive sub-cultures in one year.

Example 2

Microtuberogenic shoots of Example 1, in the rapid proliferation stage, were transferred into a high-temperature (30°C) growth chamber (other culture conditions were identical with those for the given microtuberogenic shoot proliferation) for one week, and were then moved into a low-temperature (10°C) growth chamber in complete darkness for another week. After low-temperature treatment, the microtuberogenic shoots were inoculated onto microtuber induction media (see Tables 2 and 4) on Petri dishes, and tightly sealed with parafilm. They were cultured in a growth chamber where the daytime temperature was maintained at 20°C and the nighttime temperature at 12°C; the photoperiod was 6 hours light and 18 hours dark. The light intensity was about 500 lux.

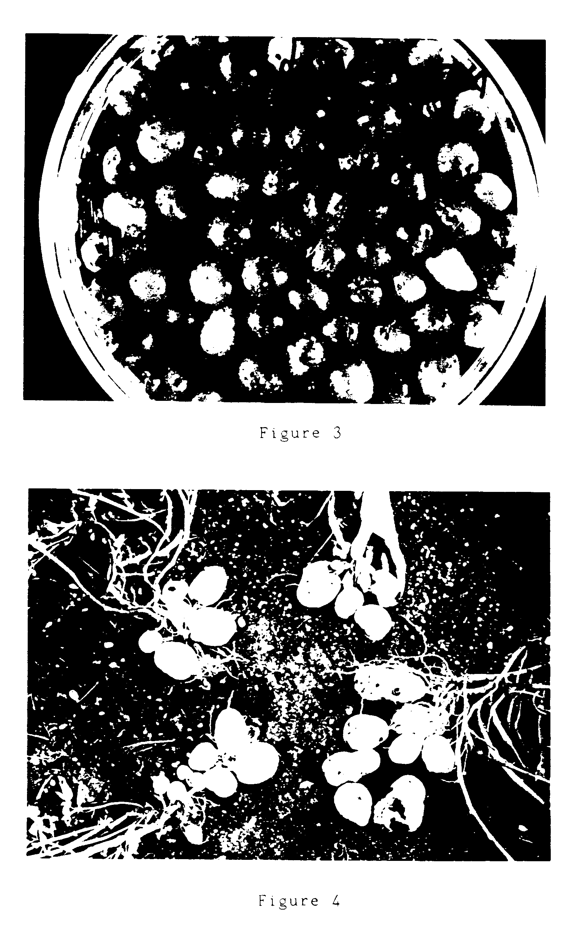

In order to maximise the use of space in the growth chamber, the Petri dishes were piled on top of each other. In most cases, about 10 days after they were transferred into such microtuber induction conditions, potato microtubers began to be formed and, after a culture period of 40-50 days, more than 10 microtubers of the size of soybeans were formed, on average, on each Petri dish (see Fig. 2).

When a growth inhibitor such as Phosphon D, Amo-1618, B-905 (N,N-dimethylaminosuccinamic acid) or chlorocholine chloride (CCC), at a concentration of 50 ppm, was added to the culture medium, the production efficiency of microtubers was greatly increased.

A comparative study was made as to production efficiency per culture space unit by one person, which indicated the difference of results between traditional flask liquid culture method using non-microtuberogenic shoots and Petri dish solid culture method using microtuberogenic shoots developed by this invention including all other treatments for increasing microtuber induction rate. The results are shown in Tables 6 and 7.

Example 3

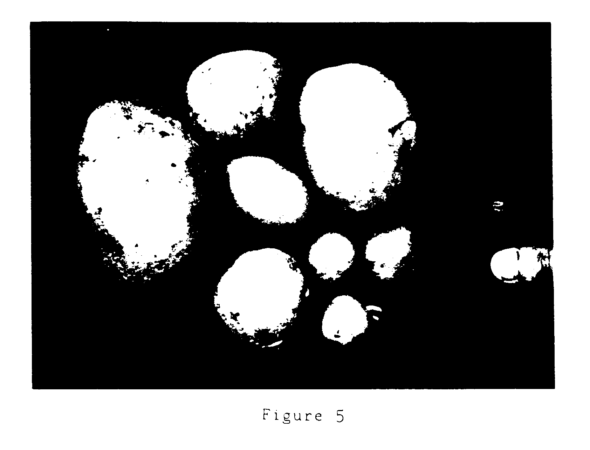

Microtubers of "Superior" species produced in quantities in Petri dishes were harvested sterile, rinsed with sterile distilled water 3 or 4 times, thus removing culture media remaining on the surface. Then they were laid out on a clean bench and dried until moisture was completely removed from the surface. After drying, the microtubers were put into empty sterile Petri dishes, tightly sealed with 3 layers of parafilm, and kept in a refrigerator at 4°C (see Fig. 3). After about 2 months, the dormancy was broken, and the microtubers readily germinated when left at room temperature for about two weeks.

When it was desired to store the microtubers for a long time without germination, they were pretreated with an abscisic acid solution (5 mg/l) for 3 hours before final low temperature storage. In this way, it was possible to store microtubers in a satisfactory condition for more than a year, and it was confirmed that the ability to germinate was not lost or harmed (see Table 9).

In order to induce germination soon after harvesting, it was possible to break dormancy by with gibberellic acid treatment (optionally with prior bathing at 38°C) (see Table 8). This technique was also used to shorten the period required for germination in the case of microtubers whose dormancy was already broken (see Table 10).

Example 4

An experiment was conducted, to compare the harvest raised by natural seed potatoes with that raised by potato microtubers. When the length of sprouts of germinating microtubers reached 2-3 mm, after germination as described in Example 3, microtubers were seeded directly on the soil. In the early stage, the growth of microtubers was rather poor compared with that of natural seed potatoes but, after the middle stage, they grew rapidly. At harvest, 3 months after seeding, microtubers grew two-thirds as tall above the soil as natural seed potatoes. Final yield per plant was also about 60-70% for the former with respect to the latter: the microtuber-derived plants produced about 507 g potatoes per plant, while the natural seed potatoes gave about 812 g per plant (see Table 11 and Figs. 4 and 5). However, since the plants derived from microtubers were much smaller than those derived from natural seed potatoes, more dense plantation should make it possible to increase yield per plantation area.

Table 1

| The composition of media used in the induction and mass proliferation of microtuberogenic shoots of 'Superior' species of potato. | |
| --- | --- |
| Composition | Content (mg/liter) |
| Ammonium nitrate | 2,000.000 |
| Potassium nitrate | 2,500.000 |
| Calcium chloride $2H_2O$ | 440.000 |
| Magnesium sulfate $7H_2O$ | 370.000 |
| Potassium Phosphate | 170.000 |
| Disodium EDTA | 37.250 |
| Ferrous sulfate $7H_2O$ | 27.850 |
| Manganese sulfate $H_2O$ | 16.900 |
| Boric acid | 6.200 |
| Zinc sulfate $7H_2O$ | 8.600 |
| Potassium iodide | 0.830 |
| Sodium molybdate $2H_2O$ | 0.250 |
| Copper sulfate $5H_2O$ | 0.025 |
| Cobalt chloride $6H_2O$ | 0.025 |
| Staba vitamins complex | See the composition in table 2. |
| Myo-inositol | 100.000 |
| Ascorbic acid | 50.000 |
| Gibberellic Acid (GA) | 0.100 |
| Zeatin riboside | 0.100 |
| Sucrose | 20,000.000 |
| Agar | 10,000.000 |
| Medium pH = 5.7 | |

Table 2

| The composition of staba vitamins complex added to the media used for the formation and rapid mass propagation of microtuberogenic shoots of 'Superior' species of potato. | |
| --- | --- |
| Composition | Content/liter |
| Cyanocobalmin | 1.5 mg |
| Folic acid | 0.5 mg |
| Riboflavin | 0.5 mg |
| Biotin | 1.0 mg |
| Choline chloride | 1.0 mg |
| Calcium pantothenate | 1.0 mg |
| Thiamine HCl | 1.0 mg |
| Nicotinamide | 2.0 mg |
| Pyriodoxine HCl | 2.0 mg |
| Para-aminobenzoic acid | 0.5 mg |

Table 3

| The effect of in vitro layering technique using petridish in tissue culture of 'Superior' species of potato on increase of the number of axillary shoots | |
| --- | --- |
| Method of culture * | Number of axillary shoots |
| Culture in flask of 250 ml | 4 ± 1.6 ** |
| Culture in petridish | 13 ± 3.5 |

\* : One initial shoot (3cm long) was inoculated, and the number of axillary shoots was counted after 4 weeks of culture.

\*\* : Mean ± S.D. of 20 cultures of each treatment.

Table 4

| The composition of the media for mass production of potato microtuber of 'Superior' species | |
|---|---|
| Composition | Content (mg/liter) |
| Ammonium nitrate | 1,000.000 |
| Potassium nitrate | 1,500.000 |
| Calcium Chloride 2$H_2$O | 440.000 |
| Magnesium sulfate 7$H_2$O | 370.000 |
| Potassium Phosphate | 500.000 |
| Disodium EDTA | 37.250 |
| Ferrous sulfate 7$H_2$O | 27.850 |
| Manganese sulfate $H_2$O | 16.900 |
| Boric acid | 6.200 |
| Zinc sulfate 7$H_2$O | 8.600 |
| Potassium iodide | 0.830 |
| Sodium molybdate 2$H_2$O | 0.250 |
| Copper sulfate 5$H_2$O | 0.025 |
| Cobalt chloride 6$H_2$O | 0.025 |
| Staba vitamins complex | See the composition in Table 2 |
| Myo-inositol | 100.000 |
| Ascorbic acid | 50.000 |
| Chloro choline chloride(CCC) | 100.000 |
| Zeatin riboside | 0.100 |
| Sucrose | 90,000.000 |
| Agar | 10,000.000 |
| Medium pH = 5.7 | |

Table 5

| A comparison of microtuber production efficiency by culture environment conditions between microtuberogenic shoots and normal (non-microtuberogenic) shoots in 'Superior' species of potato. | |
| --- | --- |
| | Number of microtubers produced per Petridish |
| In case normal shoots were cultured in normal culture condition * | 0 |
| In case normal shoots were cultured in microtuber induction condition ** | 2 ± 1.2 |
| In case microtuberogenic shoots were cultured in normal culture condition | 2 ± 0.7 |
| In case microtuberogenic shoots were cultured in microtuber induction condition | 10 ± 3.5 |

* : Culture temperature was constant at 25°C with 16 hour light and 8 hour dark photoperiod. Media used were the same as those used in microtuberogenic shoot proliferation.

** : As for media, microtuber induction media were used, and shoots were given pre-treatment at high temperature (30°C) and at low temperature in darkness (10°C), and culture temperature was varied during daytime and (at 20°C) nighttime (at 12°C). Photoperiod was 6 hour light and 18 hour dark. Light intensity was about 500 lux.

Table 6. A comparison of production efficiency of potato microtubers per culture space unit by 2 different culture method in 'Superior' species

| Culture method | Number of microtubers ** produced per culture space unit * | Production efficiency between cuture method |
| --- | --- | --- |
| a. Flask culture method *** (using liquid media) . | 385 ± 42.2 **** | $\dfrac{b}{a} = 31.9$ |
| b. Petridish culture method ***** (using solid media) | 12,280 ± 981.4 | |

* : Culture shelf of 120cm in length, 70cm in width, and 30cm in height was used.

** : This refers to microtubers more than 5mm in diameter and more than 100mg in weight, capable of actually being used as seed potatoes when seeded on soil.

*** : Erlenmeyer flask of 250ml were used.

**** : Mean ± S.D of 3 replications.

***** : Petridishes of 10cm in diameter and 1.5cm in height were used.

Table 7

| A detaild comparison of production efficiency of potato microtubers by culture method presented in Table 6 in 'Superior' species | | | |
|---|---|---|---|
| Culture method | Average number of microtubers produced per a culture vessel | Culture period required for microtuber production | Number of culture vessels to be placed per culture space unit |
| a. Flask culture method<br>b. Petrish culture method | 5<br>10 | 14-16 weeks<br>8-10 weeks | 1<br>10 |
| comparison of detailed production efficiency | 2 times | 1.5 time | 10 times |
| Comparison of total production efficiency | 2 x 1.5 x 10 = about 30 times * | | |

\* : This indicates that petrish culture method developed by this invention has production efficiency of about 30 times as high as conventional flask culture method.

Table 8

| The effect of the period of low temperature storage and gibberellic acid treatment * on dormancy breaking of potato microtubers of 'Superior' species. | | |
|---|---|---|
| Period of low-temperature storage after harvesting | Germination rate(%) ** | Germination rate in case of gibberellic acid treatment (%) |
| 1 week | 0 | 5 |
| 2 weeks | 0 | 20 |
| 3 weeks | 10 | 60 |
| 4 weeks | 25 | 90 |
| 5 weeks | 50 | 95 |
| 6 weeks | 80 | 97 |
| 7 weeks | 95 | 95 |
| 8 weeks | 95 | 95 |

\* : Treated at the concentration of 5 ppm at room temperature for 1 hour.
\*\* : Germination rate was examined after 2 weeks at room temperature.

Table 9

| The effect of abscisic acid treatment * on the inhibition of germination of potato microtubers of 'Superior' species during low temperature storage. | |
|---|---|
| Treatment | Inhibition period |
| Low-temperature storage without ABA treatment | 6 months |
| Low-temperature storage with ABA treatment | 12 months |

\* : Treated at the concentration of 10ppm at room temperature for 3 hours.

Table 10.  The effect  of warm-temperature bathing($38\,°C$,  1  hour)  and gibberellic  acid treatment (5ppm, room temperature,  1 hour) on the stimulation of germination of potato microtubers of 'Superior' species whose dormancy was already broken.

| Treatment | Period necessary for more than 90% of microtubers *  to germinate ** |
|---|---|
| No treatment | 14 days |
| GA treatment | 7 days |
| $38\,°C$ bathing treatment | 10 days |
| GA + $38\,°C$ bathing treatment | 4 days |

\*   :  Microtubers whose dormancy was already broken

\*\*  :  This refers to germination state in which sprouts are more than 1mm long and recognizable with the naked eye.

Table 11

| A comparison of per-plant yield * raised by potato microtubers and that by natural seed potatoes of 'Superior' species | |
|---|---|
| Treatment | Average yield |
| Potato microtubers | 507 ± 156.5 g |
| Natural seed potatoes | 812 ± 230.8 g |

\* : This indicates the average yield obtained from 30 potato plants each from microtubers and natural seed potatoes.

**Claims**

1. A process for the production of virus-free potato microtubers, which comprises inducing microtuberogenic shoots from virus-free potatoes to proliferate in a culture medium; treating the shoots in the light; and then culturing the treated shoots in a solid microtuber induction medium; characterised in that the culture medium has the composition given in Table 1, and that the shoots are treated in the light at an elevated temperature, followed by treatment in the dark at a low temperature.

2. A process according to claim 1, wherein the axillary shoots are substantially horizontal.

3. A process according to claim 1 or claim 2, wherein the culturing is conducted in a Petri dish.

4. A process according to any preceding claim, wherein the treatment is carried out for 1 week each at the elevated temperature and the low temperature.

5. A process according to any preceding claim, wherein the elevated temperature and the low temperature are respectively about 30°C and about 10°C.

6. A process according to any preceding claim, wherein the culturing is carried out at about 20°C in the light and at about 12°C in the dark, alternately.

7. A process according to claim 6, wherein the culturing is carried out for 6 hours at 20°C in the light, and for 18 hours at 12°C in the dark, alternately.

8. A process according to claim 6 or claim 7, wherein the intensity of the light is about 500 lux.

9. A process according to any preceding claim, wherein the potato is of the "Superior" species or Solenum tuberosum L. solenaceae.

10. A process according to any preceding claim, wherein the potato has the characteristics of that derived from the cell line deposited under the accession number ATCC 40769.

11. A process according to any preceding claim, wherein the induction medium has the composition given in Table 4 or that medium in which the chlorocholine chloride is substituted by 2,4-dichlorobenzyltributylphosphonium chloride, 2-isopropyl-4-dimethylamino-5-methylphenyl-1-piperidinecarboxylate methyl chloride or succinic acid 2,2-dimethylhydrazide.

**Patentansprüche**

1. Verfahren zur Erzeugung von Virus-freien Kartoffel-Mikroknollen, welches umfaßt, daß Mikroknollen-entwickelnde Triebe von Virus-freien Kartoffeln zur Proliferation in einem Kulturmedium induziert werden; die Triebe im Licht behandelt werden; und anschließend die behandelten Triebe in einem festen Mikroknollen-Induktionsmedium gezüchtet werden; dadurch gekennzeichnet, daß das Kulturmedium die in Tabelle 1 angegebene Zusammensetzung aufweist und daß die Triebe im Licht bei erhöhter Temperatur behandelt werden, gefolgt von einer Behandlung im Dunkeln bei niedriger Temperatur.

2. Verfahren nach Anspruch 1, worin die blattachselständigen Triebe im wesentlichen horizontal sind.

3. Verfahren nach Anspruch 1 oder Anspruch 2, worin die Züchtung in einer Petrischale durchgeführt wird.

4. Verfahren nach irgendeinem der vorhergehenden Ansprüche, worin die Behandlung jeweils 1 Woche lang bei der erhöhten Temperatur und bei der niedrigen Temperatur durchgeführt wird.

5. Verfahren nach irgendeinem der vorhergehenden Ansprüche, worin die erhöhte Temperatur und die niedrige Temperatur etwa 30°C bzw. etwa 10°C betragen.

6. Verfahren nach irgendeinem der vorhergehenden Ansprüche, worin die Züchtung abwechselnd bei etwa 20°C im Licht und bei etwa 12°C im Dunkeln durchgeführt wird.

7. Verfahren nach Anspruch 6, worin die Züchtung abwechselnd 6 Stunden lang bei 20°C im Licht und 18 Stunden lang bei 12°C im Dunkeln durchgeführt wird.

8. Verfahren nach Anspruch 6 oder Anspruch 7, worin die Intensität des Lichts etwa 500 Lux beträgt.

9. Verfahren nach irgendeinem der vorhergehenden Ansprüche, worin die Kartoffel von der Spezies "Superior" oder Solenum tuberosum L. solenaceae ist.

10. Verfahren nach irgendeinem der vorhergehenden Ansprüche, worin die Kartoffel die charakteristischen Merkmale derjenigen aufweist, die von der unter der Hinterlegungsnummer ATCC 40769 hinterlegten Zellinie stammt.

11. Verfahren nach irgendeinem der vorhergehenden Ansprüche, worin das Induktionsmedium die in Tabelle 4 angegebene Zusammensetzung aufweist oder das Medium ist, in dem das Chlorcholinchlorid durch 2,4-Dichlorbenzyltributylphosphoniumchlorid, 2-Isopropyl-4-dimethylamino-5-methylphenyl-1-piperidincarboxylatmethylchlorid oder Bernsteinsäure-2,2-dimethylhydrazid ersetzt ist.

**Revendications**

1. Procédé pour la production de microtubercules de pomme de terre exempts de virus, qui comprend l'induction d'une multiplication de pousses microtuberculogéniques à partir de pommes de terre exemptes de virus, dans un milieu de culture; le traitement des pousses à la lumière; et ensuite la culture des pousses traitées dans un milieu solide d'induction de microtubercules; caractérisé en ce que le milieu de culture a la composition donnée au Tableau 1, et en ce que les pousses sont traitées à la lumière à une température élevée, et ensuite traitées dans l'obscurité à basse température.

2. Procédé selon la revendication 1, dans lequel les pousses axillaires sont essentiellement horizontales.

3. Procédé selon la revendication 1 ou la revendication 2, dans lequel la culture est menée dans une boîte de Petri.

4. Procédé selon l'une quelconque des revendications précédentes, dans lequel le traitement est mené pendant 1 semaine à la température élevée et une semaine à la basse température.

5. Procédé selon l'une quelconque des revendications précédentes, dans lequel la température élevée et la basse température sont respectivement d'environ 30°C et d'environ 10°C.

6. Procédé selon l'une quelconque des revendications précédentes, dans lequel la culture est menée en alternance à environ 20°C à la lumière et à environ 12°C dans l'obscurité.

7. Procédé selon la revendication 6, dans lequel la culture est menée en alternance pendant 6 heures à 20°C à la lumière, et pendant 18 heures à 12°C dans l'obscurité.

8. Procédé selon la revendication 6 ou la revendication 7, dans lequel l'intensité de la lumière est d'environ 500 lux.

9. Procédé selon l'une quelconque des revendications précédentes, dans lequel la pomme de terre est de l'espèce "Superior" ou Solenum tuberosum L. solenaceae.

10. Procédé selon l'une quelconque des revendications précédentes, dans lequel la pomme de terre a les caractéristiques de celle dérivée de la souche de cellules déposée sous le numéro de matricule ATCC 40769.

11. Procédé selon l'une quelconque des revendications précédentes, dans lequel le milieu d'induction a la composition donnée au Tableau 4, ou est ce milieu dans lequel le chlorure de chlorocholine est remplacé par le chlorure de 2,4-dichlorobenzyltributylphosphonium, le chlorure de 2-isopropyl-4-diméthylamino-5-méthylphényl-1-pipéridinecarboxylate de méthyle ou le 2,2'-diméthylhydrazide de l'acide succinique.

Figure 1

Figure 2

Figure 3

Figure 4

14

Figure 5